# EUROPEAN PATENT APPLICATION

(11) **EP 1 717 581 A1**
(43) Date of publication of application: **02.11.2006**
(21) Application number: 05710069.5
(22) Date of filing: 10.02.2005
(51) Int. Cl.: G01N 30/00

(54) **METHOD OF CHEMICAL SUBSTANCE SEPARATION**

(30) Priority: 16.02.2004 JP 2004037589
(71) Applicant: Tokyo University of Agriculture and Technology Tlo Co., Ltd., Tokyo 184-8588 (JP)
(72) Inventor: CHIBA, Kazuhiro, c/o Agriculture Department, Fuchu-shi, Tokyo 1838509 (JP)
(74) Representative: Bublak, Wolfgang
(86) International application number: PCT/JP2005/002017
(87) International publication number: WO 2005/078430

(57) **Abstract**

The present invention provides a method of separating substances utilizing a solid phase wherein a substance fixed to a solid phase can be easily separated without the application of a chemical process, biochemical process, exposure to light, electrical excitation, or the like. Disclosed is a method of separating a reaction product generated by the reaction of a first substance and a second substance comprising the steps of: (a) mixing the first substance with a temperature-sensitive carrier residing in a liquid-phase state; (b) fixing an anchor region of the first substance to the temperature-sensitive carrier by converting the temperature-sensitive carrier to a solid-phase state by changing the temperature of the reaction system; (c) generating a reaction product by reacting the second substance with a reaction region of the first substance that is fixed to the temperature-sensitive carrier; (d) removing impurities from the reaction system; and (e) releasing the anchor region of the reaction product from the temperature-sensitive carrier by converting the temperature-sensitive carrier to a liquid-phase state by changing the temperature of the reaction system.

## Description

### TECHNICAL FIELD

The present invention relates to a method of separating chemical substances. More particularly, the present invention relates to a method of efficiently separating chemical substances using an interaction between a specific temperature-sensitive carrier and a substance having an anchor region.

### BACKGROUND ART

The chemical reactions or interactions of substances in chemical or biochemical processes are frequently carried out in solution. In such processes, an operation that separates desired from undesired substances is required in order to efficiently obtain the target substance at the highest possible purity. Solid-phase synthesis methods and various types of chromatography involving solid carrier particles have heretofore been widely used to carry out the separation of substances. For example, in affinity chromatography, a high-affinity ligand is distinguished and separated from other molecules by its specific binding to a probe molecule that has been chemically attached beforehand to the surface of a solid-phase carrier. This method does provide easy separation of the substance attached to the solid phase from substances dissolved in the liquid phase, but it also requires the implementation of a certain treatment in order to cleave the substance from the solid phase and obtain the target substance. That is, after the substance has been captured on a surface, a specific chemical treatment, biochemical treatment, exposure to light, or physical treatment, e.g., electrical excitation, is necessary in order to release the substance again (Seikagaku (Biochemistry), Conn and Stumpf. Third Edition, page 133, Tokyo Kagaku Dojin).

### DISCLOSURE OF THE INVENTION

An object of the present invention is to provide a method of separating substances utilizing a solid phase, wherein a substance fixed to the solid phase can be easily separated without the application of a chemical process, biochemical process, exposure to light, electrical excitation, or the like.

The present invention enables control of the capture and release of a specific substance by utilizing an interaction between a temperature-sensitive carrier and the anchor region of an anchor region-bearing substance.

A first embodiment of the present invention is a method of separating a reaction product generated by the reaction of a first substance and a second substance, comprising the steps of: (a) mixing the first substance with a temperature-sensitive carrier residing in a liquid-phase state; (b) fixing an anchor region of the first substance to the temperature-sensitive carrier by converting the temperature-sensitive carrier to a solid-phase state by changing the temperature of the reaction system; (c) generating a reaction product by reacting the second substance with a reaction region of the first substance that is fixed to the temperature-sensitive carrier; (d) removing impurities from the reaction system; and (e) releasing the anchor region of the reaction product from the temperature-sensitive carrier by converting the temperature-sensitive carrier to a liquid-phase state by changing the temperature of the reaction system, wherein the first substance has an anchor region capable of being fixed to the temperature-sensitive carrier and a reaction region that reacts with the second substance, and wherein the temperature-sensitive carrier is reversibly changed from a solid-phase state to a liquid-phase state by a change in temperature, which fixes the anchor region in the solid-phase state and does not fix the anchor region in the liquid-phase state.

A second embodiment of the present invention is a method of separating a reaction product generated by the reaction of a first substance and a second substance, comprising the steps of: (a) reacting the first substance with the second substance to generating a reaction product; (b) mixing the reaction product with a temperature-sensitive carrier residing in a liquid-phase state; (c) fixing an anchor region of the reaction product to the temperature-sensitive carrier by converting the temperature-sensitive carrier to a solid-phase state by changing the temperature of the reaction system; (d) removing impurities from the reaction system; and (e) releasing the anchor region of the reaction product from the temperature-sensitive carrier by converting the temperature-sensitive carrier to a liquid-phase state by changing the temperature of the reaction system, wherein the first substance has an anchor region capable of being fixed to the temperature-sensitive carrier and a reaction region that reacts with the second substance, the anchor region is introduced into the reaction product through the reaction between the first and second substances, and wherein the temperature-sensitive carrier is reversibly changed from a solid-phase state to a liquid-phase state by a change in temperature, which fixes the anchor region in the solid-phase state and does not fix the anchor region in the liquid-phase state.

A third embodiment of the present invention is a method of separating a complex generated by the interaction of a first substance and a second substance, comprising the steps of: (a) mixing the first substance with a temperature-sensitive carrier residing in a liquid-phase state; (b) fixing an anchor region of the first substance to the temperature-sensitive carrier by converting the temperature-sensitive carrier to a solid-phase state by changing the temperature of the reaction system; (c) generating a complex by interacting the second substance with an interaction region of the first substance that is fixed to the temperature-sensitive carrier; (d) removing impurities from the reaction system; and (e) releasing the anchor region of the complex from the temperature-sensitive carrier by converting the temperature-sensitive carrier to a liquid-phase state by changing the temperature of the reaction system, wherein the first substance has an anchor region capable of being fixed to the temperature-sensitive carrier and an interaction region that can interact with the second substance, and wherein the temperature-sensitive carrier is reversibly changed from a solid-phase state to a liquid-phase state by a change in temperature, which fixes the anchor region in the solid-phase state and does not fix the anchor region in the liquid-phase state.

A fourth embodiment of the present invention is a method of separating a complex generated by the interaction of a first substance and a second substance, comprising the steps of: (a) interacting the first substance with the second substance to generate a complex; (b) mixing the complex with a temperature-sensitive carrier residing in a liquid-phase state; (c) fixing an anchor region of the complex to the temperature-sensitive carrier by converting the temperature-sensitive carrier to a solid-phase state by changing the temperature of the reaction system; (d) removing impurities from the reaction system; and (e) releasing the anchor region of the complex from the temperature-sensitive carrier by converting the temperature-sensitive carrier to a liquid-phase state by changing the temperature of the reaction system, wherein the first substance has an anchor region capable of being fixed to the temperature-sensitive carrier and has an interaction region that interacts with the second substance, the anchor region is introduced into the complex through the interaction between the first and second substances, and wherein the temperature-sensitive carrier is reversibly changed from a solid-phase state to a liquid-phase state by a change in temperature, which fixes the anchor region in the solid-phase state and does not fix the anchor region in the liquid-phase state.

### BRIEF DESCRIPTION OF THE DRAWINGS

Figure 1 shows the temperature-induced conversion of a temperature-sensitive carrier between a solid-phase state and a liquid-phase state;
Figure 2 shows a temperature-sensitive carrier adsorbed to the surface of a solid-phase carrier;
Figure 3 shows a temperature-sensitive carrier in micelle or emulsion form;
Figure 4 shows a method of separating a complex generated by interaction;
Figure 5 shows a temperature-sensitive micellar carrier;
Figure 6 shows peptide synthesis; and
Figure 7 shows peptide synthesis.

### PREFERRED EMBODIMENT OF THE INVENTION

The temperature-sensitive carrier used in the present invention has the properties of reversible conversion to a liquid-phase state from a solid-phase state upon a change in temperature. The temperature-sensitive carrier has the ability in the solid-phase state to fix the anchor region of the first substance, and the inability in the liquid-phase state to fix the anchor region. There are no particular limitations on the temperature-sensitive carrier, as long as it has these properties, and the temperature-sensitive carrier can be exemplified by hydrocarbons. Among the hydrocarbons, C₁₀₋₃₀ hydrocarbons are preferred and normal-tetradecane, normal-hexadecane, normal-octadecane, eicosane, and cyclohexane are more preferred. Moreover, by varying the carbon chain of these hydrocarbons, the temperature of conversion from a solid-phase state to a liquid-phase state can be changed as desired. The temperature of conversion from a solid-phase state to a liquid-phase state can also be changed by intermixing two or more hydrocarbons.

While these hydrocarbons are converted to a liquid-phase state from a solid-phase state by raising the temperature, substances may be used that in contrast undergo conversion to a solid-phase state from a liquid-phase state when the temperature is raised.

As used herein, "liquid-phase state" encompasses, in addition to the liquid phase, semi-solid states as well as states in which the temperature-sensitive carrier has become softened. As used herein, "solid-phase state" encompasses, in addition to the solid phase, states in which the temperature-sensitive carrier has become hardened. Thus, a hardened state assumed by the temperature-sensitive carrier has the ability to fix the anchor region, while a softened or semi-solid state assumed by the temperature-sensitive carrier does not have the ability to fix the anchor region.

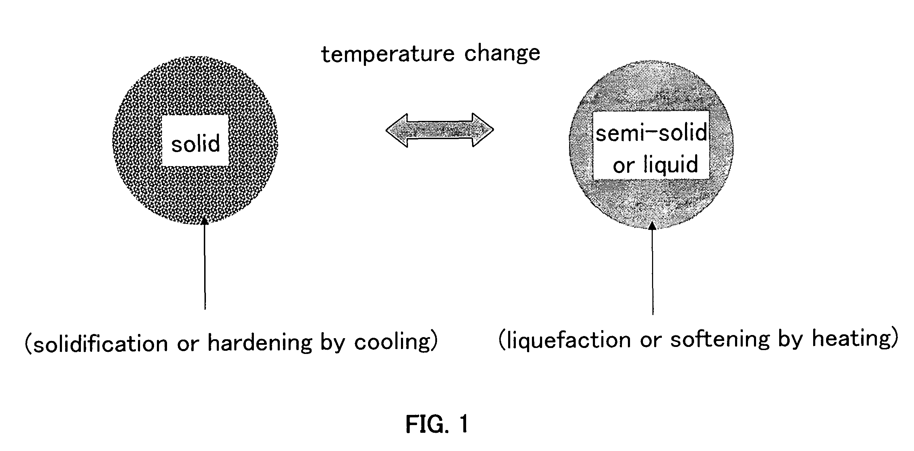

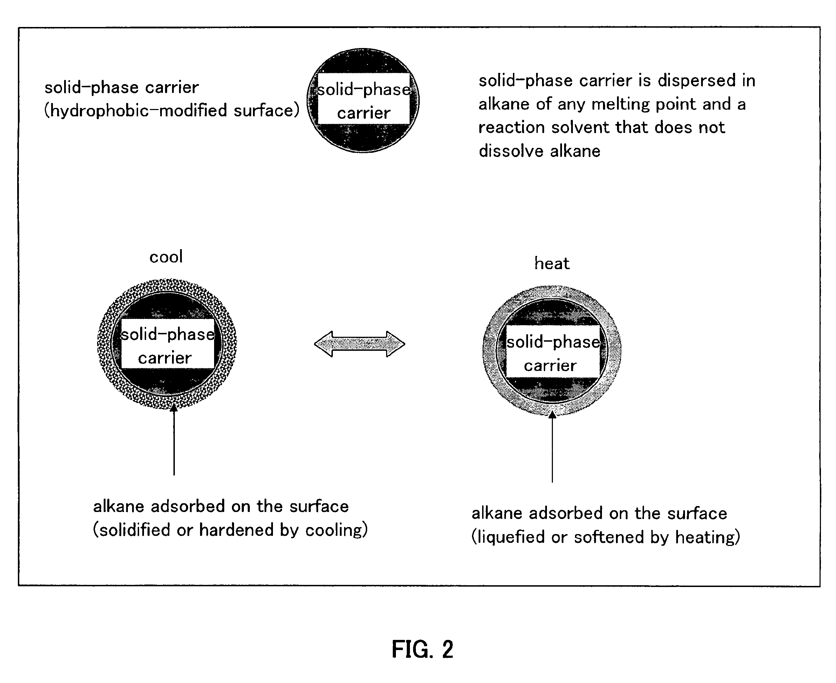

The temperature-sensitive carrier used in the present invention may have, as shown in Figure 1, a configuration in which the temperature-sensitive carrier as a whole reversibly changes from a solid-phase state to a liquid-phase state, or, as shown in Figure 2, may have a configuration in which the temperature-sensitive carrier is adsorbed or attached to the surface of another solid carrier. The solid carrier, as used herein, is a solid carrier that can adsorb or attach the temperature-sensitive carrier, but is not otherwise particularly limited, and can be exemplified by silica gel, glass beads and platinum powder having octadecyl groups attached to the surface thereof (ODS), and so forth. In addition, taking cyclohexane as an example, the carrier can also be the material obtained by dissolving alkane with a higher melting point than cyclohexane in cyclohexane and then cooling the mixture of the alkane with cyclohexane and inducing precipitation.

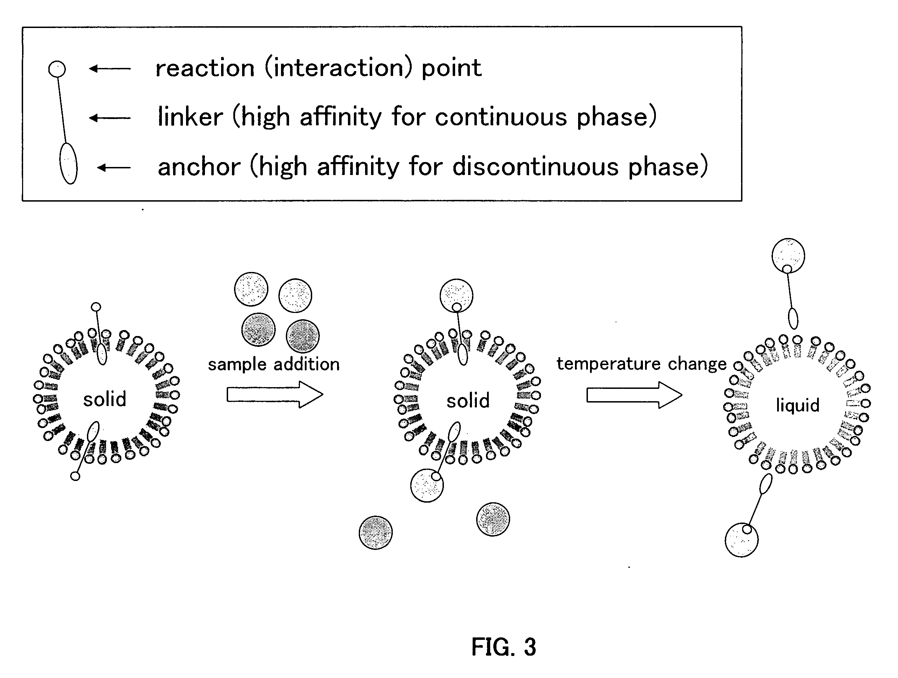

The temperature-sensitive carrier as used herein may also have, as shown in Figure 3, the configuration of an emulsion or micelle surrounded by surfactant. The surfactant as used herein is a surfactant that has the capacity to form a micelle or emulsion, but is not otherwise particularly limited, and can be exemplified by phospholipids such as phosphatidylcholine and lysophosphatidylcholine. Suitable mixtures of two or more of these surfactants may also be used.

The first substance as used herein contains an anchor region capable of being fixed to the temperature-sensitive carrier and a reaction region that reacts with the second substance or an interaction region that interacts with the second substance. The anchor region has the ability to become fixed to the temperature-sensitive carrier, but is not otherwise particularly limited, and can be exemplified by long-chain alkyl groups, polyether chains such as polyethylene glycol (PEG), and polymer chains such as polystyrene and polyethylene.

The reaction region of the first substance reacts with the second substance, but is not otherwise particularly limited, and can be exemplified by an amino group, a carboxyl group, an aldehyde group, a sulfonic acid group, a hydroxyl group, a thiol group, halogenated alkyl groups, unsaturated hydrocarbyl groups, a nitro group, acid anhydrides, acid halides, an isocyanate group, an isothiocyanate group, and so forth.

The interaction region of the first substance interacts with the second substance, but is not otherwise particularly limited, and can be exemplified by avidin, biotin, antigens, antibodies, physiologically active substances, peptides, oligosaccharides, glycopeptides, nucleic acids, the epitopes of peptides or proteins, and so forth.

The first substance as used herein can be produced by attaching an anchor region as described above to a reaction region or interaction region. A linker region may optionally be placed between the anchor region and the reaction or interaction region. The linker region functions to maintain a suitable distance between the anchor region and the reaction or interaction region and thereby functions to facilitate reaction or interaction with the second substance. There are no particular limitations on the linker region, and it can be exemplified by long-chain alkyl groups, polyether chains such as polyethylene glycol (PEG), polythioether chains, and so forth.

There are no particular limitations on the method of producing the first substance, and it can be obtained by the usual chemical syntheses or other procedures.

The second substance as used herein reacts with the reaction region of the first substance, but is not otherwise particularly limited, and can be exemplified by substances that contain, for example, a carboxyl group, an amino group, an aldehyde group, a sulfonic acid group, a hydroxyl group, a thiol group, halogenated alkyl, unsaturated hydrocarbyl, a nitro group, acid anhydride, acid halide, an isocyanate group, or an isothiocyanate group. Or, the second substance interacts with the interaction region of the first substance, but is not otherwise particularly limited, and can be exemplified by biotin, avidin, antigens, antibodies, physiologically active substances, peptides, oligosaccharides, glycopeptides, nucleic acids, the epitopes of peptides or proteins, and so forth.

For example, when peptide synthesis is being carried out, the reaction region of the first substance can be an amino group, while the second substance can be an amino acid in which the amino group is protected by t-BOC and the carboxyl group has been activated by, for example, diisopropylcarbodiimide (DIPCD).

The first embodiment of the present invention is described in the following.

The first embodiment of the present invention is a method of separating a reaction product generated by the reaction of a first substance and a second substance, comprising the steps of: (a) mixing the first substance with a temperature-sensitive carrier residing in a liquid-phase state; (b) fixing an anchor region of the first substance to the temperature-sensitive carrier by converting the temperature-sensitive carrier to a solid-phase state by changing the temperature of the reaction system; (c) generating a reaction product by reacting the second substance with a reaction region of the first substance that is fixed to the temperature-sensitive carrier; (d) removing impurities from the reaction system; and (e) releasing the anchor region of the reaction product from the temperature-sensitive carrier by converting the temperature-sensitive carrier to a liquid-phase state by changing the temperature of the reaction system, wherein the first substance has an anchor region capable of being fixed to the temperature-sensitive carrier and a reaction region that reacts with the second substance, and wherein the temperature-sensitive carrier is reversibly changed from a solid-phase state to a liquid-phase state by a change in temperature, which fixes the anchor region in the solid-phase state and does not fix the anchor region in the liquid-phase state.

In the step (a) cited above, the first substance and the temperature-sensitive carrier are uniformly dissolved or dispersed. In this case, the first substance and the temperature-sensitive carrier may be dissolved or dispersed using a suitable solvent, such as water. Then, in step (b), the temperature-sensitive carrier is converted to a solid-phase state by changing the temperature, whereby the anchor region of the first substance becomes fixed to the temperature-sensitive carrier. In step (c), a reaction product fixed to the anchor region is obtained by reacting the reaction region of the first substance with the second substance. A reaction promoter and/or a reaction activator may be used in this step. In step (d), impurities, such as unreacted second substance, reaction promoter, reaction activator, and so forth, are removed. The removal procedure can be exemplified by washing the reaction system with solvent and filtration. The reaction product can be easily separated from the impurities at this point because the anchor region of the reaction product is fixed to the temperature-sensitive carrier. When the temperature-sensitive carrier has a small size, separation for the purpose of separation from the solvent or impurities dissolved in the solvent may be carried out by centrifugal separation utilizing the difference in specific gravity between the temperature-sensitive carrier and the solvent. Or, a substance with a high affinity for a specific substance may be preliminarily attached to or captured on the surface of the temperature-sensitive carrier, and the small temperature-sensitive solid particles may then be fixed to a specific solid surface using a separate solid surface that interacts with this region of affinity or using interaction with a polymer. This enables facile separation even in those cases where there is no difference in specific gravity between the solvent and the temperature-sensitive solid particles. In step (e), the temperature-sensitive carrier is brought into a liquid-phase state and the reaction product is thereby released from the temperature-sensitive carrier. The reaction product can be released at this point without carrying out a chemical reaction or other procedures. The target substance can be obtained by recovery of the thus released reaction product.

For example, when peptide synthesis is to be carried out, a peptide with a desired amino acid length can be synthesized by following step (d) with repeated executions of steps (c) and (d) using an elongating amino acid as the second substance.

The second embodiment of the present invention is explained below.

A second embodiment of the present invention is a method of separating a reaction product generated by the reaction of a first substance and a second substance, comprising the steps of: (a) reacting the first substance with the second substance to generate a reaction product; (b) mixing the reaction product with a temperature-sensitive carrier residing in a liquid-phase state; (c) fixing an anchor region of the reaction product to the temperature-sensitive carrier by converting the temperature-sensitive carrier to a solid-phase state by changing the temperature of the reaction system; (d) removing impurities from the reaction system; and (e) releasing the anchor region of the reaction product from the temperature-sensitive carrier by converting the temperature-sensitive carrier to a liquid-phase state by changing the temperature of the reaction system, wherein the first substance has an anchor region capable of being fixed to the temperature-sensitive carrier and a reaction region that reacts with the second substance, the anchor region is introduced into the reaction product through the reaction between the first and second substances, and wherein the temperature-sensitive carrier is reversibly changed from a solid-phase state to a liquid-phase state by a change in temperature, which fixes the anchor region in the solid-phase state and does not fix the anchor region in the liquid-phase state.

In step (a) as described above, the first and second substances are reacted to generate a reaction product. For example, a reaction promoter, reaction activator, and so forth, may be used in this step. This reaction can be run in a homogeneous system in solvent, and the reaction can therefore be more efficient than in the prior solid-phase synthesis methods. Then, in step (b), the reaction product and a temperature-sensitive carrier are uniformly dissolved or dispersed. In step (c), the temperature is changed to convert the temperature-sensitive carrier to a solid-phase state, whereby the anchor region of the reaction product is fixed to the temperature-sensitive carrier. In step (d), impurities, such as unreacted second substance, reaction promoter, reaction activator, and so forth, are removed. The removal procedure can be exemplified by washing the reaction system with solvent and filtration. The reaction product is easily separated from the impurities at this point because the anchor region of the reaction product is fixed to the temperature-sensitive carrier. In step (e), the temperature-sensitive carrier is brought into a liquid-phase state, thereby releasing the reaction product from the temperature-sensitive carrier. The reaction product can be released at this point without carrying out a chemical reaction or other procedures. The target substance can be obtained by recovery of the released reaction product.

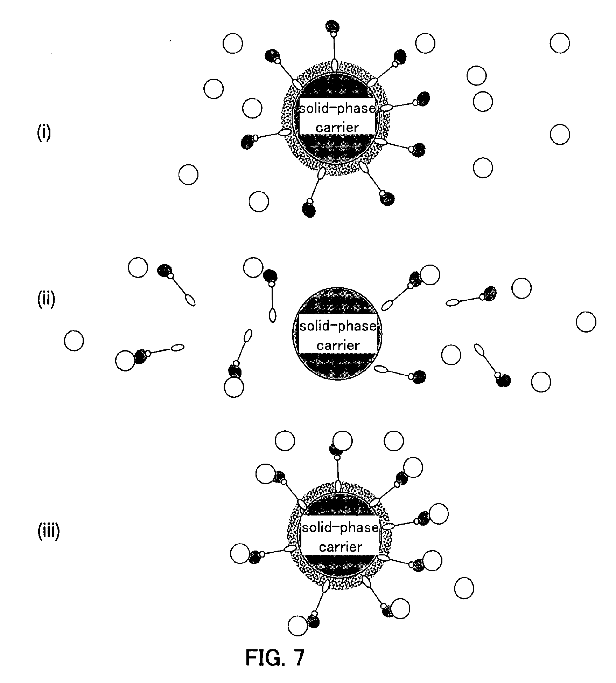

For example, when peptide synthesis is to be carried out, a peptide with a desired amino acid length can be synthesized by following step (e) with repeated executions of steps (a) to (e) using an elongating amino acid as the second substance. A schematic diagram of peptide synthesis is shown in Figure 7. In Figure 7(i), for example, cyclohexane is retained and solidified on a solid-phase (ODS) surface dispersed in DMF at 0°C. At this point, a C-terminal amino acid attached to an anchor molecule is retained along with the cyclohexane solidified on the surface of the solid-phase carrier. In Figure 7(ii), for example, the cyclohexane at the solid-phase surface is completely dissolved in DMF by heating to 50°C, and the C-terminal amino acid attached to the anchor molecule is also dissolved in the DMF phase. At this point, a reaction with an amino acid elongation reagent occurs in a homogeneous solution. In Figure 7(iii), upon recooling to 0°C while stirring, the cyclohexane and anchor molecule dissolved in the DMF become strongly retained on the ODS surface due to solidification of the cyclohexane. In this state, excess amino acid elongation reagent can be washed away and removed using DMF.

The third embodiment of the present invention is described below.

The third embodiment of the present invention is a method of separating a complex generated by the interaction of a first substance and a second substance, comprising the steps of: (a) mixing the first substance with a temperature-sensitive carrier residing in a liquid-phase state; (b) fixing an anchor region of the first substance to the temperature-sensitive carrier by converting the temperature-sensitive carrier to a solid-phase state by changing the temperature of the reaction system; (c) generating a complex by interacting the second substance with an interaction region of the first substance that is fixed to the temperature-sensitive carrier; (d) removing impurities from the reaction system; and (e) releasing the anchor region of the complex from the temperature-sensitive carrier by converting the temperature-sensitive carrier to a liquid-phase state by changing the temperature of the reaction system, wherein the first substance has an anchor region capable of being fixed to the temperature-sensitive carrier and an interaction region that can interact with the second substance, and wherein the temperature-sensitive carrier is reversibly changed from a solid-phase state to a liquid-phase state by a change in temperature, which fixes the anchor region in the solid-phase state and does not fix the anchor region in the liquid-phase state.

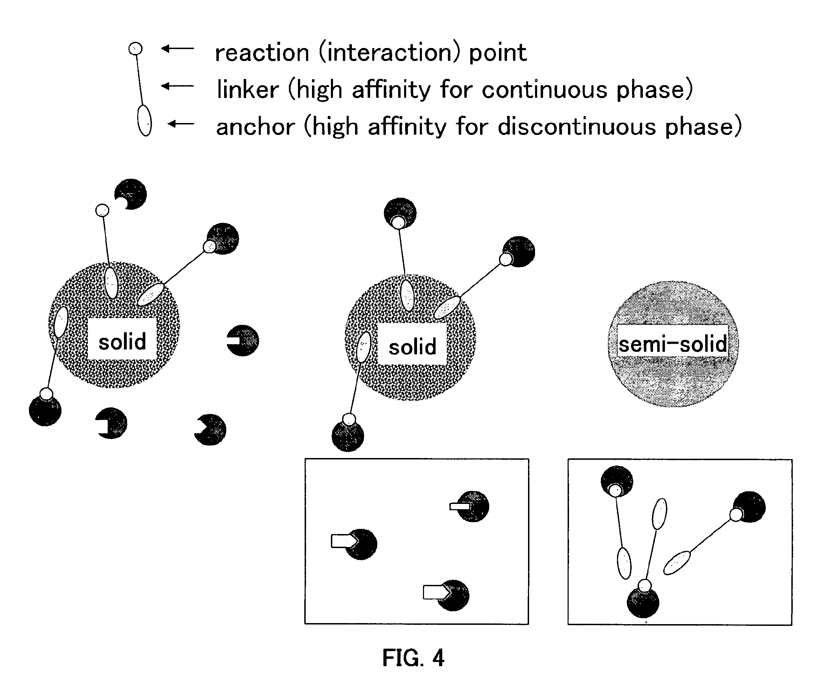

In the step (a), the first substance and the temperature-sensitive carrier are uniformly dissolved or dispersed. In this case, the first substance and the temperature-sensitive carrier may be dissolved or dispersed using a suitable solvent, such as water. Then, in step (b), the temperature-sensitive carrier is converted to a solid-phase state by changing the temperature, whereby the anchor region of the first substance becomes fixed to the temperature-sensitive carrier. In step (c), a complex fixed to the anchor region is generated by interacting the interaction region of the first substance with the second substance. During this step, other substances that do not interact with the first substance may be dissolved or dispersed as such in the solvent without forming a complex. In step (d), impurities, such as the other substances that do not interact with the first substance, are removed. The removal procedure can be exemplified by washing the reaction system with solvent and filtration. The complex can be easily separated from the impurities at this point because the anchor region of the complex is fixed to the temperature-sensitive carrier. In step (e), the temperature-sensitive carrier is brought into a liquid-phase state and the complex is thereby released from the temperature-sensitive carrier. The complex can be released at this point without carrying out a chemical reaction or other procedures. The target substance can be obtained by recovery of the released complex (see Figure 4).

The fourth embodiment of the present invention is described in the following.

The fourth embodiment of the present invention is a method of separating a complex generated by the interaction of a first substance and a second substance, comprising the steps of: (a) interacting the first substance with the second substance to generate a complex; (b) mixing the complex with a temperature-sensitive carrier residing in a liquid-phase state; (c) fixing an anchor region of the complex to the temperature-sensitive carrier by converting the temperature-sensitive carrier to a solid-phase state by changing the temperature of the reaction system; (d) removing impurities from the reaction system; and (e) releasing the anchor region of the complex from the temperature-sensitive carrier by converting the temperature-sensitive carrier to a liquid-phase state by changing the temperature of the reaction system, wherein the first substance has an anchor region capable of being fixed to the temperature-sensitive carrier and has an interaction region that interacts with the second substance, the anchor region is introduced into the complex through the interaction between the first and second substances, and wherein the temperature-sensitive carrier is reversibly changed from a solid-phase state to a liquid-phase state by a change in temperature, which fixes the anchor region in the solid-phase state and does not fix the anchor region in the liquid-phase state.

In step (a) as described above, the first and second substances are interacted to obtain a complex. During this step, other substances that do not interact with the first substance will not form a complex. Then, in step (b), the complex and a temperature-sensitive carrier are uniformly dissolved or dispersed. In this case, the complex and the temperature-sensitive carrier may be dissolved or dispersed using a suitable solvent, such as water. In step (c), the temperature is changed to convert the temperature-sensitive carrier to a solid-phase state, whereby the anchor region of the complex is fixed to the temperature-sensitive carrier. In step (d), impurities, such as the other substances that do not interact with the first substance, are removed. The removal procedure can be exemplified by washing the reaction system with solvent and filtration. The complex is easily separated from the impurities at this point because the anchor region of the complex is fixed to the temperature-sensitive carrier. In step (e), the temperature-sensitive carrier is brought into a liquid-phase state, thereby releasing the complex from the temperature-sensitive carrier. The complex can be released at this point without carrying out a chemical reaction or other procedures. The target substance can be obtained by recovery of the released complex.

### Example 1

### <Synthesis of an ligand-bearing anchor molecule>

Biotin (0.11 g), N,N'-disucciimidyl carbonate (0.12 g), and triethylamine (0.24 g) were dissolved in 15 mL of DMF and stirred for 6 hours at room temperature. 8-Amino-3,6-dioxaoctanamine cholesteryl carbamate (0.26 g) was then added and the reaction solution was stirred for 16 hours. The solvent was distilled off under reduced pressure on a rotary evaporator followed by separation and purification by silica gel column chromatography to give the ligand-bearing anchor molecule, {3-[2-(2-{2-[5-(2-oxohexahydrothieno[3,4-d]imidazol-4-yl)pentanoylamino]ethoxy}ethoxy)ethoxy]ethyl}carbamic acid 13-(1,5-dimethylhexyl)-10-methyl-2,3,4,7,8,9,10,11,12,13,14,15,16,17-tetradecahydro-1H-cyclopenta[a]phenanthren-3-yl ester, at 60% yield.

### <Preparation of a temperature-sensitive micellar carrier>

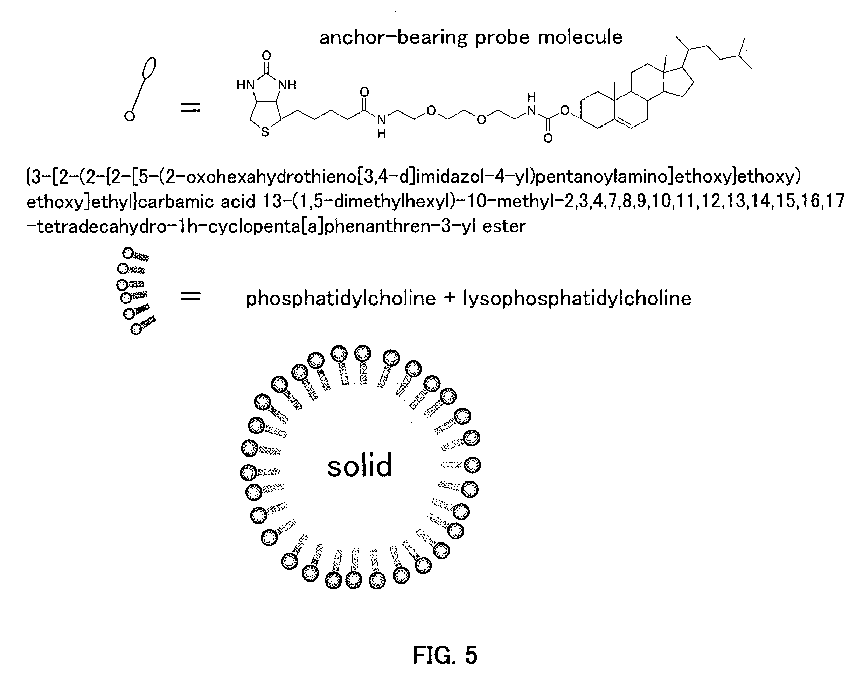

7.5 mg of phosphatidylcholine, 5.0 mg of lysophosphatidylcholine, 30 mg of normal-hexadecane, and 35.0 mg of the ligand-bearing anchor molecule, {3-[2-(2-{2-[5-(2-oxohexahydrothieno[3,4-d]imidazol-4-yl)pentanoylamino]ethoxy}ethoxy)ethoxy]ethyl}carbamic acid 13-(1,5-dimethylhexyl)-10-methyl-2,3,4,7,8,9,10,11,12,13,14,15,16,17-tetradecahydro-1H-cyclopenta[a]phenanthren-3-yl ester, were dissolved in 2 mL of water, and stirred for 3 minutes at 20,000 rpm with a dual-cylinder mixer (IKA^{R}-WERKE) on a water bath at 30°C, then cooled for 10 minutes on an ice bath (see Figure 5).

### <Detection of avidin by electrophoresis>

To 20 µL of the resulting micelle solution was added 20 µL of a 6 mg/mL aqueous solution of avidin (ImmunoPure^{R} Avidin, PIERCE) followed by stirring for 1 minute with a homomixer (IKA^{R}-WERKE). Then, while continuously cooling the sample, the following procedure was carried out five times: washing by the addition of buffer (TrisHCl buffer + EDTA) and concentration using a Micro-con YM-100 (Microcon^{R} centrifugal filter, MW 100,000, MILLIPORE). The reaction solution was then heated for 1 hour while stirring on a water bath at 40°C. The micelles were thereafter removed by centrifugal separation (Chibitan II desktop centrifuge, MILLIPORE); the lower phase aqueous solution was sampled; and electrophoresis was carried out under the following conditions.
electrophoresis chamber: XV PANTERA SYSTEM ERICA
electrophoresis gel: XV PANTERA GEL
stain: Bio-Safe™ Coomassie; Commasie^{R} G250 Stain (BIO-RAD™)
buffer: 0.0625 M TrisHCl (pH 6.8), 5% 2-mercaptoethanol, 0.005% BPB, 20% glycerol, 2% SDS

4 µL of sample was diluted with 4 µL of sample buffer followed by denaturation by heating for 15 minutes at 90°C. The electrophoresis gel was washed with MilliQ water and 6 µL each of the sample was applied to the lanes; current (200V) was applied for 17 minutes. At the end of electrophoresis, the gel was washed three times with MilliQ water, soaked in the staining solution for one hour, and then immersed in MilliQ water overnight for destaining. In this way, the avidin molecule captured on the micelle surface by interaction with the ligand anchor molecule was separated and detected.

### Example 2

### <Temperature-sensitive, homogeneous solution reaction (interaction) - Construction of solid-phase surface capture system (1)>

2.0 gram of ODS (silica gel with the octadecyl group attached on the surface) was dispersed in a solution prepared by mixing 1 mL of cyclohexane and 9 mL of dimethylformamide (DMF) at 25°C. The mixture was stirred at the same temperature using a vortex mixer. The mixture became separated into two phases, where cyclohexane was the main component in the upper layer and was supported on the ODS surface. When this solution was cooled to 0°C, ODS became dispersed in DMF and cyclohexane on the ODS surface froze and solidified in a surface-supported state. When this dispersion was heated to 50°C, the cyclohexane melted and was dissolved homogeneously in DMF. These phenomena could be reversibly repeated by changing the temperature.

### <Peptide bond formation using cyclohexane-DMF-ODS system>

The temperature-sensitive, homogeneous solution reaction (interaction) - solid-phase surface capture system based on DMF described above was heated to 50°C and 0.1 mmol of 3,4,5-trisoctadecyloxybenzyl 2-amino-3-methylbutyric acid was then dissolved therein. A 20 mL of DMF solution containing 0.3 mmol of Fmoc-Gly-OBt and 0.5 mmol of diisopropylcarbodiimide (DIPCD) was added and stirred for 90 minutes. The reaction system was then cooled to 0°C with stirring. After cooling, the DMF solution was removed by suction filtration and the solid (cyclohexane-ODS) was washed three times with 20 mL of DMF cooled to 5°C or below. The solid (cyclohexane-ODS) was then heated to 40°C and the cyclohexane was distilled off under reduced pressure using a vacuum pump. The ODS was finally washed with acetonitrile. From the acetonitrile solution, 3,4,5-trisoctadecyloxybenzyl 2-[2-(9H-fluoren-9-ylmethoxycarbonylamino)acetylamino]-3-methylbutyiric acid was obtained at 97% yield.
¹H-NMR (400 MHz) δ: 7.77 (2H, d, J=7.3 Hz), 7.59 (2H, d, J=7.3 Hz), 7.40 (2H, t, J=7.3 Hz), 7.31 (2H, dt, J=0.7, 7.3 Hz), 6.52 (2H, s), 6.38 (1H, d, J=8.4 Hz), 5.44-5.37 (1H, br), 5.10 (1H, d, J=12.1 Hz), 5.02 (1H, d, J=12.1 Hz), 4.62 (2H, dd, J=8.4, 4.8 Hz), 4.42 (2H, d, J=7.0 Hz), 4.24 (1H, t, J=7.0 Hz), 3.96-3.92 (8H, m), 2.21-2.16 (1H, m), 1.81-1.76 (4H, m), 1.75-1.70 (2H, m), 1.48-1.43 (6H, m), 1.37-1.21 (84H, br), 0.91 (3H, d, J=7.0 Hz), 0.88 (9H, t, J=7.0 Hz), 0.86 (3H, d, J=7.0 Hz); ¹³C-NMR (150 MHz) δ: 171.5, 168.7, 156.5, 153.1, 143.6, 141.2, 138.3, 130.0, 127.7, 127.0, 125.0, 120.0, 107.0, 73.4, 69.2, 67.5, 67.4, 57.1, 47.1, 32.0, 31.4, 30.4, 29.8, 29.7, 29.5, 29.4, 26.1, 22.8, 19.0, 17.7, 14.2; MALDI TOF-MS(pos) calcd. for:C₈₃H₁₃₈N₂O₈ [M+Na]⁺ 1314, found: 1314.

### Example 3

### <Temperature-sensitive, homogeneous solution reaction (interaction) - Construction of a solid-phase surface capture system (2)>

2.0 gram of ODS (silica gel with the octadecyl group attached on the surface) was dispersed in a solution prepared by mixing 1 mL of cyclohexane and 7 mL of N-methyl-2-pyrrolidone (NMP) at 20°C. The mixture was stirred at the same temperature using a vortex mixer. The mixture became separated into two phases, where cyclohexane was the main component in the upper layer and was supported on the ODS surface. When this solution was cooled to -10°C, the cyclohexane on the ODS surface froze and solidified in a surface-supported state. When this dispersion was heated to 20°C, the cyclohexane melted and was dissolved homogeneously in NMP. These phenomena could be reversibly repeated by changing the temperature.

### <Peptide bond formation using cyclohexane-NMP-ODS system>

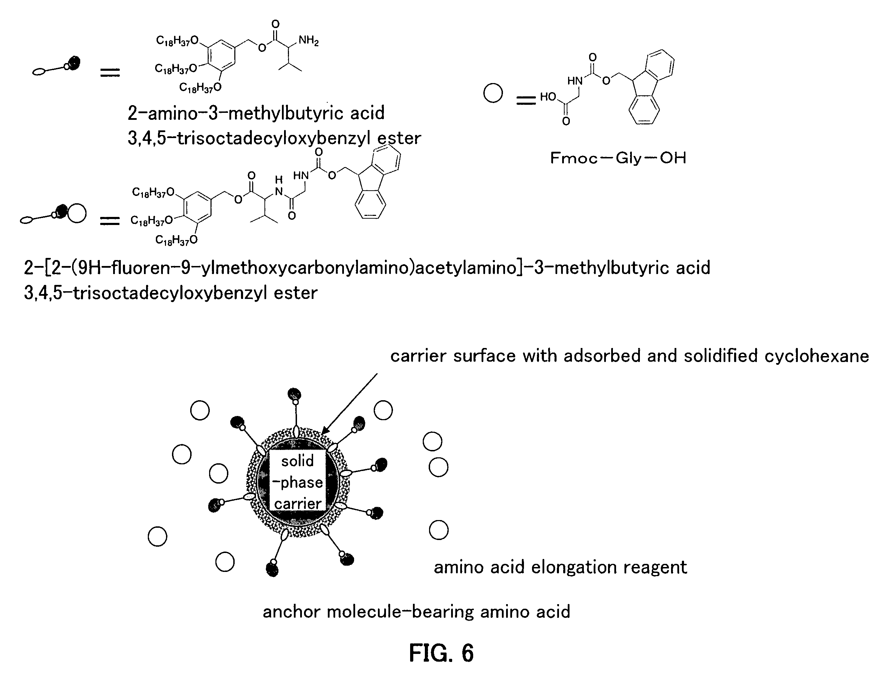

The temperature-sensitive, homogeneous solution reaction (interaction) - solid-phase surface capture system based on NMP described above was heated to 20°C and 0.1 mmol of 3,4,5-trisoctadecyloxybenzyl 2-amino-3-methylbutyric acid was then dissolved therein. 10 mL of an NMP solution containing 0.3 mmol of Fmoc-Gly-OBt and 0.5 mmol of diisopropylcarbodiimide (DIPCD) was added and stirred for 90 minutes. The reaction system was then cooled to -10°C with stirring. After cooling, the NMP solution was removed by suction filtration and the solid (cyclohexane-ODS) was washed three times with 20 mL of NMP cooled to 5°C or below. The solid (cyclohexane-ODS) was then heated to 20°C and the cyclohexane was distilled off under reduced pressure using a vacuum pump. The ODS was finally washed with acetonitrile. From the acetonitrile solution, 3,4,5-trisoctadecyloxybenzyl 2-[2-(9H-fluoren-9-ylmethoxycarbonylamino)acetylamino]-3-methylbutyric acid was obtained at 99% yield (see Figure 6).

### Example 4

### <Continuous sequential peptide bond formation using cyclohexane-DMF-ODS system>

The temperature-sensitive, homogeneous solution reaction (interaction) - solid-phase surface capture system based on DMF described above was heated to 50°C and 0.1 mmol of 3,4,5-trisoctadecyloxybenzyl 2-amino-3-methylbutyric acid was then dissolved therein. A 20 mL of DMF solution containing 0.3 mmol of Fmoc-Gly-OBt and 0.5 mmol of diisopropylcarbodiimide (DIPCD) was added and stirred for 90 minutes. The reaction system was then cooled to 0°C with stirring. After cooling, the DMF solution was removed by suction filtration and the solid (cyclohexane-ODS) was washed three times with 20 mL of DMF. The solid (cyclohexane-ODS) was then heated to 20°C and 10 mL of a 10% DBU/DMF solution was added. After stirring for 2 minutes at the same temperature, the reaction solution was cooled to 0°C with stirring. After the completion of cooling and solidification, ODS was filtered again and washed with DMF. The Fmoc group attached to the N-terminal amino group was cleaved by the DBU treatment step, resulting in conversion to the amino group. Repetition of this same process enabled sequential peptide bond formation.
¹H-NMR (600 MHz) δ: 7.74 (1H, d, J=9.2 Hz), 6.53 (2H, s), 5.11 (1H, d, J=12.1 Hz), 5.02 (1H, d, J=12.1 Hz), 4.61 (1H, dd, J=9.2, 5.1 Hz), 3.95 (4H, t, J=6.6 Hz), 3.94 (2H, t, J=6.6 Hz), 3.39 (2H, s), 2.24-2.18 (1H, m), 1.81-1.76 (4H, m), 1.75-1.71 (2H, m), 1.49-1.44 (6H, m), 1.37-1.20 (84H, br), 0.93 (3H, d, J=7.0 Hz), 0.90-0.86 (12H, m); ¹³C-NMR (150 MHz) δ: 172.6, 171.8, 153.1, 130.3, 125.5, 106.9, 73.4, 69.2, 67.2, 56.6, 44.8, 32.0, 31.3, 30.4, 30.3, 29.8, 29.7, 29.5, 29.4, 26.2, 22.8, 19.1, 17.8, 14.2; MALDI TOF-MS(pos) calcd. for:C₆₈H₁₂₈N₂O₆ [M+Na]⁺ 1091, found:1091.

### Example 5

### <Continuous sequential peptide bond formation using cyclohexane-NMP-ODS system>

The temperature-sensitive, homogeneous solution reaction (interaction) - solid-phase surface capture system based on NMP described above was heated to 20°C and 0.1 mmol of 3,4,5-trisoctadecyloxybenzyl 2-amino-3-methylbutyric acid was then dissolved therein. A 10 mL of NMP solution containing 0.3 mmol of Fmoc-Gly-OBt and 0.5 mmol of diisopropylcarbodiimide (DIPCD) was added and stirred for 90 minutes. The reaction system was then cooled to -10°C with stirring. After cooling, the NMP solution was removed by suction filtration and the solid (cyclohexane-ODS) was washed three times with 20 mL of NMP cooled to 5°C or below. The solid (cyclohexane-ODS) was then heated to 20°C and 10 mL of a 10% DBU/NMP solution was added. After stirring for 2 minutes at the same temperature, the reaction solution was cooled to -10°C with stirring. After the completion of cooling and solidification, ODS was filtered again and washed with NMP. The Fmoc group attached to the N-terminal amino group was cleaved by the DBU treatment step, resulting in conversion to the amino group. Repetition of this same process enabled sequential peptide bond formation.

### Example 6

### <Continuous sequential peptide bond formation using cyclohexane-DMF-eicosane (alkane)>

0.1 mmol of 3,4,5-trisoctadecyloxybenzyl 2-amino-3-methylbutyric acid and 100 mg of eicosane were dissolved in 9 mL of DMF and 1 mL of cyclohexane. A 5 mL of DMF solution containing 0.3 mmol of Fmoc-Gly-OBt and 0.5 mmol of diisopropylcarbodiimide (DIPCD) was added and stirred for 90 minutes at 25°C. The reaction system was then cooled to -10°C with stirring. At this time, white solid particles precipitated. The DMF solution was removed by suction filtration and the solid (cyclohexane-eicosane) was washed three times with 20 mL of DMF cooled to 0°C or below. The solid (cyclohexane- eicosane) was then heated to 20°C and 10 mL of a 10% DBU/DMF solution was added. After stirring for 2 minutes at the same temperature, the reaction solution was cooled to -10°C with stirring. After the completion of cooling and solidification, eicosane was filtered again and washed with DMF cooled to 0°C or below. The Fmoc group attached to the N-terminal amino group was cleaved by the DBU treatment step, resulting in conversion to the amino group. Repetition of this same process enabled sequential peptide bond formation.

### Example 7

### <Application as a scavenger that removes an amino group-containing substance from a homogeneous solution system (use of ODS particles) (1)>

0.1 mmol of 3,4,5-trisoctadecyloxybenzaldehyde and 3 mL of cyclohexane were dissolved at 50°C in a 10 mL of DMF solution containing 0.05 mmol of aniline. The solution was stirred for 16 hours, 2.0 g ODS was added, and cooled to - 10°C with stirring. After cooling, the DMF solution and the solid (cyclohexane-ODS) were separated by suction filtration. While cooling, the solid fraction was washed with 10 mL of DMF cooled to 0°C or below. In this way, aniline was reacted with aldehyde and captured in the solid at 98% yield.

### Example 8

### <Application as a scavenger that removes an amino group-containing substance from a homogeneous solution system (without the use of ODS particles) (2)>

0.1 mmol of 3,4,5-trisoctadecyloxybenzaldehyde and 3 mL of cyclohexane were dissolved at 50°C in a 10 mL of NMP solution containing 0.05 mmol of aniline. The solution was stirred for 16 hours and then cooled to -10°C with stirring. After cooling, the NMP solution and the solid (cyclohexane) were separated by suction filtration. While cooling, the solid fraction was washed with 10 mL of NMP. In this way, aniline was reacted with aldehyde and captured in the solid at 96% yield.

### Example 9

### <Application as a scavenger that removes an amino group-containing substance from a homogeneous solution system (without the use of ODS particles) (3)>

0.1 mmol of 3,4,5-trisoctadecyloxybenzaldehyde, 3 mL of cyclohexane, and 500 mg of eicosane were dissolved at 50°C in a 10 mL of DMF solution containing 0.05 mmol of aniline. The solution was stirred for 16 hours and then cooled to 0°C with stirring. After cooling, the DMF solution and the solid (mixture of cyclohexane and eicosane) were separated by suction filtration. While cooling, the solid fraction was washed with 10 mL of DMF. In this way, aniline was reacted with aldehyde and captured in the solid at 98% yield.

### Reference Example

### <Production of a temperature-sensitive solid-phase carrier>

300 mg of normal-hexadecane and 35.0 mg of the anchor-bearing probe molecule, {3-[2-(2-{2-[5-(2-oxohexahydrothieno[3,4-d]imidazol-4-yl)pentanoylamino] ethoxy} ethoxy)ethoxy] ethyl} carbamic acid 13-(1,5 - dimethylhexyl)-10-methyl-2,3,4,7,8,9,10,11,12,13,14,15,16,17-tetradecahydro-1H-cyclopenta[a]phenanthren-3-yl ester, were dissolved in 2 mL of water and stirred for 3 minutes at 20,000 rpm with a dual-cylinder mixer (IKA^{R}-WERKE) on a water bath at 30°C. The container was then quenched to 0°C with stirring in the same manner. This resulted in the dispersion and solidification of a temperature-sensitive carrier in water, with the probe moiety of the anchor-bearing probe molecule being oriented toward the aqueous phase.

### INDUSTRIAL APPLICABILITY

The present invention not only provides a substantial improvement in the efficiency of continuous synthesis/purification/separation processes for chemical substances and biochemical substances, but also enables innovative developments in a very broad range of technical fields, for example, in methods of substance separation and production that utilize interactions between biomolecules and in methods for detecting/analyzing specific substances.

## Claims

1. A method of separating a reaction product generated by reaction of a first substance and a second substance, comprising the steps of:
(a) mixing the first substance with a temperature-sensitive carrier residing in a liquid-phase state;
(b) fixing an anchor region of the first substance to the temperature-sensitive carrier by converting the temperature-sensitive carrier to a solid-phase state by changing temperature of a reaction system;
(c) generating a reaction product by reacting the second substance with a reaction region of the first substance that is fixed to the temperature-sensitive carrier;
(d) removing impurities from the reaction system; and
(e) releasing the anchor region of the reaction product from the temperature-sensitive carrier by converting the temperature-sensitive carrier to a liquid-phase state by changing temperature of the reaction system,
wherein the first substance has an anchor region capable of being fixed to the temperature-sensitive carrier and a reaction region that reacts with the second substance, and
wherein the temperature-sensitive carrier is reversibly changed from a solid-phase state to a liquid-phase state by a change in temperature, which fixes the anchor region in the solid-phase state and does not fix the anchor region in the liquid-phase state.

2. A method of separating a reaction product generated by reaction of a first substance and a second substance, comprising the steps of:
(a) reacting the first substance with the second substance to generate a reaction product;
(b) mixing the reaction product with a temperature-sensitive carrier residing in a liquid-phase state;
(c) fixing an anchor region of the reaction product to the temperature-sensitive carrier by converting the temperature-sensitive carrier to a solid-phase state by changing temperature of a reaction system;
(d) removing impurities from the reaction system; and
(e) releasing the anchor region of the reaction product from the temperature-sensitive carrier by converting the temperature-sensitive carrier to a liquid-phase state by changing temperature of the reaction system,
wherein the first substance has an anchor region capable of being fixed to the temperature-sensitive carrier and a reaction region that reacts with the second substance, and the anchor region is introduced into the reaction product through the reaction between the first and second substances, and
wherein the temperature-sensitive carrier is reversibly changed from a solid-phase state to a liquid-phase state by a change in temperature, which fixes the anchor region in the solid-phase state and does not fix the anchor region in the liquid-phase state.

3. A method of separating a complex generated by interaction of a first substance and a second substance, comprising the steps of:
(a) mixing the first substance with a temperature-sensitive carrier residing in a liquid-phase state;
(b) fixing an anchor region of the first substance to the temperature-sensitive carrier by converting the temperature-sensitive carrier to a solid-phase state by changing temperature of a reaction system;
(c) generating a complex by interacting the second substance with an interaction region of the first substance that is fixed to the temperature-sensitive carrier;
(d) removing impurities from the reaction system; and
(e) releasing the anchor region of the complex from the temperature-sensitive carrier by converting the temperature-sensitive carrier to a liquid-phase state by changing temperature of the reaction system,
wherein the first substance has an anchor region capable of being fixed to the temperature-sensitive carrier and an interaction region that can interact with the second substance, and
wherein the temperature-sensitive carrier is reversibly changed from a solid-phase state to a liquid-phase state by a change in temperature, which fixes the anchor region in the solid-phase state and does not fix the anchor region in the liquid-phase state.

4. A method of separating a complex generated by interaction of a first substance and a second substance, comprising the steps of:
(a) interacting the first substance with the second substance to generate a complex;
(b) mixing the complex with a temperature-sensitive carrier residing in a liquid-phase state;
(c) fixing an anchor region of the complex to the temperature-sensitive carrier by converting the temperature-sensitive carrier to a solid-phase state by changing temperature of a reaction system;
(d) removing impurities from the reaction system; and
(e) releasing the anchor region of the complex from the temperature-sensitive carrier by converting the temperature-sensitive carrier to a liquid-phase state by changing temperature of the reaction system,
wherein the first substance has an anchor region capable of being fixed to the temperature-sensitive carrier and an interaction region that interacts with the second substance, the anchor region is introduced into the complex through the interaction between the first and second substances, and
wherein the temperature-sensitive carrier is reversibly changed from a solid-phase state to a liquid-phase state by a change in temperature, which fixes the anchor region in the solid-phase state and does not fix the anchor region in the liquid-phase state.
